# EUROPÄISCHE PATENTSCHRIFT

(11) **EP 2 145 788 B1**
(45) Veröffentlichungstag und Bekanntmachung des Hinweises auf die Patenterteilung: **19.12.2012**
(21) Anmeldenummer: 09164918.6
(22) Anmeldetag: 08.07.2009
(51) Int. Cl.: B60H 3/00, A61L 9/12

(54) **Verfahren zur Steuerung der Duftintensität einer Beduftungsvorrichtung**
Method for controlling the aroma intensity of an odoriser device
Procédé de commande de l'intensité odorante d'un dispositif d'embaumement

(30) Priorität: 14.07.2008 DE 102008032883
(43) Veröffentlichungstag der Anmeldung: 20.01.2010
(73) Patentinhaber: Behr GmbH & Co. KG, 70469 Stuttgart (DE)
(72) Erfinder: Lochmahr, Karl, 71665 Vaihingen/Enz (DE); Rais, Thomas, 71672 Marbach/Neckar (DE); Treier, Joachim, 77728 Oppenau (DE); Liermann, Andreas, 67117 Limburgerhof (DE); Tihsler, Sigmar, 75417 Mühlacker (DE); Pitz, Eric, 70199 Stuttgart (DE)
(74) Vertreter: Grauel, Andreas

(56) Entgegenhaltungen:
- DE-A1- 10 328 747
- DE-B3- 10 301 214
- DE-C1- 19 955 035
- US-A1- 2006 222 672

## Beschreibung

Die Erfindung betrifft ein Verfahren zur Steuerung der Duftintensität bei einer Beduftungsvorrichtung. Weiterhin betrifft die Erfindung eine Steuerschaltung für eine Beduftungsvorrichtung. Darüber hinaus betrifft die Erfindung eine Beduftungsvorrichtung sowie ein Kraftfahrzeug mit einer Beduftungsvorrichtung.

Eine Beduftung der Umgebungsluft findet in unterschiedlichsten Anwendungsgebieten eine zunehmende Verbreitung. Ein derartiges Anwendungsgebiet liegt beispielsweise bei der Beduftung von Kraftfahrzeugen, insbesondere von Automobilen vor. Hier liegt der Wunsch nach einer Beduftung der Fahrzeuginnenluft in zunehmendem Ausmaß vor. Dies gilt insbesondere für bestimmte Kulturkreise, wie beispielsweise den asiatischen oder indischen Kulturkreis, in dem der Wunsch nach einer Beduftung der Fahrzeuginnenluft tendenziell stärker ausgeprägt ist, als beispielsweise im westeuropäischen Kulturkreis.

Eine traditionelle Form der Beduftung von Kraftfahrzeuginnenräumen existiert beispielsweise in Form sogenannter Duftbäume. Diese Duftbäume sind aus Karton oder einem entsprechende Material gefertigt und mit einem Duftstoff getränkt, der sukzessive an die Außenluft abgegeben wird. Duftbäume werden in der Regel am Fahrzeuginnenspiegel befestigt, wo sie gut erkennbar sind. Oftmals werden Duftbäume als kitschig und störend empfunden. Darüber hinaus geben die Duftbäume den Duftstoff kontinuierlich an die Außenluft ab, sodass sich die Fahrzeuginsassen nach einiger Zeit an den Duft gewöhnen und ihn schlussendlich nicht mehr wahr nehmen. Manche Personen bevorzugen jedoch eine dauerhaft wahrnehmbare Beduftung des Fahrzeuginnenraums. Um eine Gewöhnung an den Duftstoff zu verhindern, wurden bereits Beduftungsvorrichtungen vorgeschlagen, bei denen der Duftstoff stoßweise in Intervallen freigesetzt wird. Derartige Duftstöße können grundsätzlich über einen beliebig langen Zeitraum hinweg bewusst wahrgenommen werden.

In EP1854647A1 wird ein Beduftungssystem vorgeschlagen, bei dem die Beduftung im Impulsbetrieb erfolgt. Das heißt, eine Beduftung erfolgt für einen gewissen Zeitraum, wie beispielsweise für einen Zeitraum von 10 Sekunden. Anschließend erfolgt über eine gewisse Zeitspanne hinweg (beispielsweise über eine Zeitspanne von 1 Minute hinweg) keine Abgabe eines Duftstoffs. Alternativ zur Beduftung im Impulsbetrieb wird auch vorgeschlagen, dass eine Beduftung in Abhängigkeit von Messwerten erfolgt, die über entsprechende Duftsensoren ermittelt werden. Eine Beduftung kann dann in Abhängigkeit von der ermittelten Konzentration und/oder Güte der Luft erfolgen. Eine Beduftung in Form von Intervallen erfolgt dann jedoch nicht mehr.

In US 5,429,180 wird zur Verstärkung des Beduftungserlebnisses einer Beduftungsvorrichtung vorgeschlagen, dass die Duftstoffe intervallartig freigesetzt werden, wobei die Beduftungszyklen und/oder die beduftungsfreien Zeitdauern erratisch (zufällig) gewählt werden. Als Zufallsgenerator wird ein Zufallsgenerator von 1/f-Typ vorgeschlagen. Zusätzlich wird vorgeschlagen, dass in definierten Zyklen anstelle des hauptsächlich verwendeten ersten Duftstoffs ein zweiter Duftstoff freigesetzt wird.

In US 4,495,560 werden unterschiedliche Beduftungsvorrichtungen vorgeschlagen, bei denen unterschiedliche Parameter der Beduftungsvorrichtung mit einem Zufallssignal angesteuert werden. Beispielsweise wird vorgeschlagen, dass die Duftfreisetzungsintervalle und/oder die Lüfterstärke in Abhängigkeit von einem Zufallssignal gewählt werden, welches beispielsweise von einem Zufallsgenerator vom 1/f⁰-Typ, vom 1/f¹-Typ oder vom 1/f²-Typ erzeugt wird.

Die DE 10 328 747 A1 zeigt ein Verfahren gemäß Oberbegriff des Anspruchs 1.

Nachteilig bei Beduftungsvorrichtungen gemäß dem Stand der Technik ist insbesondere, dass sie zwar oftmals ein dauerhaft wahrnehmbares Beduftungserlebnis erzeugen können, welches gegebenenfalls durch eine zufällige Freisetzung von Duftstoffen nochmals gesteigert werden kann. Jedoch ist es mit den bisherigen Beduftungsvorrichtungen nicht möglich, ein jeweils weitgehend optimal an die konkret vorherrschenden Einsatzbedingungen angepasstes, dauerhaft wahrnehmbares Beduftungserlebnis bereit zu stellen.

Die Aufgabe der Erfindung besteht somit darin, ein gegenüber dem Stand der Technik verbessertes Verfahren zur Steuerung der Duftintensität bei einer Beduftungsvorrichtung vorzuschlagen. Darüber hinaus besteht die Aufgabe der Erfindung darin, eine verbesserte Steuerschaltung für Beduftungsvorrichtungen, eine verbesserte Beduftungsvorrichtung sowie ein verbessertes Fahrzeug vorzuschlagen.

Es wird vorgeschlagen, ein Verfahren zur Steuerung der Duftintensität bei einer Beduftungsvorrichtung derart auszubilden, dass zumindest eine Grundstellwertvorgabe durch zumindest eine Korrekturfunktion beeinflusst wird. Bei der Grundstellwertvorgabe kann es sich insbesondere um die grundsätzliche Beduftungsanforderung handeln, also beispielsweise darum, ob eine Beduftung zu einem gewissen Zeitpunkt erwünscht ist oder nicht, welche Beduftungsintensität realisiert werden soll, ob eine Beduftung dauerhaft wahrnehmbar sein soll oder nicht, welche Duftstoffe freigesetzt werden sollen und dergleichen. Diese Grundstellwertvorgabe wird dann mit Hilfe von Korrekturfunktionen an die jeweils konkret vorherrschenden Einsatzbedingungen angepasst. Beispielsweise kann eine Anpassung auf die Umweltbedingungen, auf den Einsatzort bzw. die Einsatzart und/oder auf sinnesphysiologische Eigenschaften vom Menschen hin erfolgen. Dies wird durch die Anwendung geeigneter Korrekturfunktionen realisiert. Bei einer derartigen Korrekturfunktion kann es sich um eine beliebige Art einer mathematischen Funktion, gegebenenfalls auch einer mathematischen Relation handeln. Beispielsweise ist an lineare, quadratische, trigonometrische, zyklische, rechteckförmige, dreieckförmige, sägezahnförmige und/oder parabelförmige Funktionen zu denken. Selbstverständlich ist es auch möglich, eine konstante Funktion (also einen multiplikativen und/oder einen additiven Faktor) zu verwenden. Es erfolgt also nicht (nur) eine Verstärkung der individuellen Beduftungserfahrung, was beispielsweise durch eine eratische Freisetzung von Duftstoffen realisiert werden kann. Vielmehr erfolgt eine individuelle Anpassung an die jeweils konkret vorherrschenden Einsatzbedingungen. Dies kann gegebenenfalls auch eine Verminderung der Freisetzung von Duftmitteln zur Folge haben. Dabei ist es jedoch dennoch möglich, die Anpassung an die Umgebungsbedingungen derart zu gestalten, dass eine zumindest im Wesentlichen dauerhafte Wahrnehmbarkeit der freigesetzten Duftstoffe realisiert werden kann.

Wenn eine Mehrzahl an Korrekturfunktionen verwendet wird, kann die Anpassung an die jeweils konkreten Einsatzbedingungen besonders genau verfolgen. Insbesondere ist es auch möglich, dass unterschiedliche Korrekturfunktionen unterschiedliche Aufgaben haben bzw. unterschiedliche Ziele erfolgen. So könnte beispielsweise eine Korrekturfunktion die Anpassung an Umweltbedingungen außerhalb des Fahrzeuginnenraums ermöglichen, wohingegen eine zweite Korrekturfunktion eine Anpassung an die Umweltbedingungen innerhalb des Kraftfahrzeugs ermöglicht und/oder eine dritte Korrekturfunktion die Anpassung an sinnesphysiologische Eigenschaften der Fahrzeuginsassen ermöglicht.

Erfindungsgemäß ist eine Korrekturfunktion als Basiskorrekturfunktion und eine Korrekturfunktion als Anti-Adaptionskorrekturfunktion ausgeführt. Eine Basiskorrekturfunktion berücksichtigt dabei insbesondere Faktoren die unabhängig davon zu berücksichtigen sind, ob die Beduftung von den Benutzern dauerhaft wahrnehmbar sein soll, oder nicht. Hierbei kann es sich beispielsweise um länderspezifische Geruchsgewohnheiten oder externe Geruchsereignisse handeln. Bei den externen Geruchsereignissen kann es sich beispielsweise um das Vorhandensein von Geruchsstoffen in der dem Fahrzeuginnenraum zuzuführenden Umgebungsluft handeln. Beispielsweise kann es ein unangenehmer Umgebungsgeruch erfordern, eine größere Menge an Duftstoffen freizusetzen, um den unangenehmen Außengeruch zu überdecken. Bei den Anti-Adaptionskorrekturfunktionen handelt es sich dagegen üblicherweise um Korrekturfunktionen, die die dauerhafte Wahrnehmbarkeit der freigesetzten Duftstoffe für die Benutzer, sowie gegebenenfalls auch die dauerhaft gleichförmige Wahrnehmbarkeit der Duftintensität sicher stellen.

Als besonders vorteilhaft hat es sich erwiesen, wenn wenigstens eine Korrekturfunktion der Gruppe entnommen ist, die länderspezifische Korrekturfunktionen, Außenluftkorrekturfunktionen, Kabinentermperaturkorrekturfunktionen, Luftmengenkorrekturfunktionen, Umluft-/Frischluftauswahlkorrekturfunktionen, Luftverteilungskorrekturfunktionen, Luftfeuchtekorrekturfunktionen, Sonnenlastkorrekturfunktionen, Fahrzeitkorrekturfunktionen, Betriebszeitkorrekturfunktionen, sinnesphysiologische Korrekturfunktionen, Uhrzeitkorrekturfunktionen, Fahrgeschwindigkeitskorrekturfunktionen und Fahrzeugbesetzungskorrekturfunktionen umfasst. Erste Versuche haben ergeben, dass die genannten Korrekturfunktionen einen besonders großen Einfluss haben. Unter sinnesphysiologischen Korrekturfunktionen sind insbesondere Korrekturfunktionen zu verstehen, welche sinnesphysiologische Eigenschaften von Menschen berücksichtigen. Beispielsweise bewirken einsetzender Regen, Helligkeit bzw. Dunkelheit (Tag oder Nacht, Tunneldurchfahrten), Bewölkung, Sonnenschein usw. eine unterschiedliche Duftwahrnehmung. Von den genannten Korrekturfunktionen sind üblicherweise die länderspezifischen Korrekturfunktionen, die Außenluftkorrekturfunktionen (Berücksichtigung beispielsweise von Umgebungsdüften, Umgebungsdruck), die Luftmengenkorrekturfunktionen, die Umluft-/Frischluftauswahlkorrekturfunktionen, die Luftfeuchtekorrekturfunktionen, sowie die sinnesphysiologischen Korrekturfunktionen den Basiskorrekturfunktionen zuzuordnen. Demgegenüber sind üblicherweise Kabinentemperaturkorrekturfunktionen, Luftmengenkorrekturfunktionen, Umluft-/Frischluftauswahlkorrekturfunktionen, Luftverteilungskorrekturfunktionen, Luftfeuchtekorrekturfunktionen, Sonnenlastkorrekturfunktionen, Fahrzeitkorrekturfunktionen, Betriebszeitkorrekturfunktionen, sinnesphysiologische Korrekturfunktionen, Uhrzeitkorrekturfunktionen, Fahrgeschwindigkeitskorrekturfunktionen sowie Fahrzeugsbesetzungskorrekturfunktionen den Anti-Adaptionskorrekturfunktionen zuzuordnen. Dabei ist es durchaus möglich, dass manche Korrekturfunktionen beiden Gruppen zuzuordnen sind, was im Übrigen auch erklärt, dass manche Korrekturfunktionen doppelt genannt wurden.

Als sinnvoll kann es sich weiterhin erweisen, wenn zumindest eine Grundstellwertvorgabe die vom Bediener eingestellte Duftintensität umfasst. Hier kann der Bediener beispielsweise die Beduftungsfunktion der Beduftungsvorrichtung ein- oder ausschalten. Darüber hinaus kann er eine Beduftungsintensität, beispielsweise eine schwache Beduftungsintensität, eine mittlere Beduftungsintensität sowie eine starke Beduftungsintensität auswählen. Mit einer derartigen Vorrichtung kann die Beduftung gemäß unterschiedlicher Benutzerwünsche erfolgen.

Denkbar ist es, dass eine Korrekturfunktion zumindest bereichsweise als analytische Funktion hinterlegt ist. Eine derartige Hinterlegung bietet sich insbesondere für Korrekturfunktionen an, die besonders gut durch ein mathematisches bzw. physikalisches Modell beschreibbar sind. Beispielsweise könnte es sich um additive Konstanten, Korrekturmultiplikatoren, lineare Funktionen, quadratische Funktionen, trigonometrische Funktionen und/oder parabelartige Funktionen handeln. Eine derartige Hinterlegung kann besonders einfach besonders große Wertebereiche umfassen und ist darüber hinaus auch sehr speicherfreundlich.

Es kann sich jedoch ebenfalls als vorteilhaft erweisen, zumindest eine Korrekturfunktion zumindest bereichsweise als Wertetabelle zu hinterlegen. Ein derartiges Hinterlegen ist insbesondere dann sinnvoll, wenn Korrekturen auf Versuchsergebnissen beruhen, deren Ergebnisdaten nur schwer mit analytischen Funktionen beschrieben werden können. Dies kann beispielsweise für sinnesphysiologische Korrekturfunktionen der Fall sein. Um die Anzahl der erforderlichen Messwerte zu verringern ist es selbstverständlich möglich, zwischen zwei hinterlegten Werten liegende Ergebnisse beispielsweise durch Runden anzunähern, oder Interpolationsverfahren zu verwenden. Darüber hinaus ist es möglich, in Teilbereichen (insbesondere in Bereichen, in denen die experimentellen Ergebnisse "gutmütig") sind, die entsprechenden Korrekturfunktionen in Form analytischer Funktionen (gegebenenfalls können auch bereichsweise unterschiedliche analytische Funktionen genutzt werden) zu beschreiben.

Es kann sich als sinnvoll erweisen, wenn eine Änderung der Duftintensität zumindest zeitweise und/oder zumindest teilweise durch Änderung der freigesetzten Duftmenge, durch Änderung des Luftvolumens, durch Änderung der Beduftungsintervalle und/oder durch Änderung der beduftungsfreien Intervalle erfolgt. Gerade durch eine intervallartige Beduftung ist es möglich, eine dauerhafte Wahrnehmbarkeit der Duftstoffe zu realisieren. Durch Änderung des Verhältnisses von freigesetzter Duftmenge und/oder Änderung des Luftvolumens ist es besonders gut möglich, die Menge an Duftmittel pro Luftvolumeneinheit zu variieren.

Es erweist sich als besonders sinnvoll, wenn das Verfahren zur Beduftung eines Fahrzeugs, insbesondere eines Kraftfahrzeugs, vorzugsweise eines kraftbetriebenen Landfahrzeugs verwendet wird. Bei einem Landfahrzeug kann es sich in beliebiger Weise um ein schienengebundenes und/oder ein nicht-schienengebundenes Landfahrzeug handeln. Gerade in solchen Fahrzeugen ist eine Beduftung oftmals in besonderem Maße erwünscht. Denkbar sind aber auch Einsatzfälle für Luftfahrzeuge, Wasserfahrzeuge, Gebäude und dergleichen.

Weiterhin wird eine Steuerschaltung für eine Beduftungsvorrichtung vorgeschlagen, welche derart ausgebildet und eingerichtet ist, dass sie ein Verfahren gemäß der obigen Beschreibung durchführen kann. Die Steuerschaltung weist dann die bereits genannten Vorteile und Eigenschaften in analoger Weise auf. Auch wenn es grundsätzlich möglich ist, dass die Steuerschaltung das Verfahren in beliebiger Weise durchführt (beispielsweise mechanisch durch Steuerscheiben, Hebelanordnungen und dergleichen), so ist es besonders bevorzugt, die Steuerschaltung als elektronische Steuerschaltung auszuführen. Hier sind insbesondere auch relativ komplexe Korrekturfunktionen auf relativ einfache Weise zu realisieren. Im Übrigen ist es auch möglich, dass die Steuerschaltung zusätzliche Aufgaben übernimmt. Umgedreht ist es selbstverständlich auch denkbar, dass eine bereits ohnehin vorhandene Steuerschaltung (beispielsweise ein Einplatinencomputer in einem Kraftfahrzeug) die Steueraufgaben zur Durchführung des vorgeschlagenen Beduftungsverfahrens mit übernimmt.

Besonders vorteilhaft ist es, wenn die Steuerschaltung wenigstens eine Speichereinrichtung aufweist, in welcher zumindest eine Korrekturfunktion gespeichert ist. Die Speicherung kann dabei in Form von analytischen Funktionen und/oder in Form von Wertetabellen erfolgen. Besonders vorteilhaft ist es, wenn die Speichereinrichtung als elektronische Speichereinrichtung ausgebildet ist (beispielsweise als PROM, als EPRM, als EEPROM, als Flashspeicher usw.). Selbstverständlich ist es auch möglich, dass die Speichereinrichtung mechanisch (beispielsweise in Form von Steuerscheiben), magnetisch (Festplatten, Disketten) oder optisch (CD, DVD) erfolgt.

Weiterhin wird eine Beduftungseinrichtung, insbesondere eine Beduftungseinrichtung für ein Fahrzeug, insbesondere für ein Kraftfahrzeug, vorzugsweise für ein kraftbetriebenes Landfahrzeug vorgeschlagen, welche zumindest eine Steuerschaltung mit dem oben beschriebenen Aufbau aufweist. Die Beduftungsvorrichtung weist dann die bereits im Zusammenhang mit dem Verfahren und/oder im Zusammenhang mit der Steuerschaltung genannten Eigenschaften und Vorteile in analoger Weise auf.

Schließlich wird auch ein Fahrzeug, insbesondere ein Kraftfahrzeug, vorzugsweise ein kraftbetriebenes Landfahrzeug vorgeschlagen, welches zumindest eine Beduftungsvorrichtung mit dem oben beschriebenen Aufbau und/oder zumindest eine Steuerschaltung mit dem oben beschriebenen Aufbau aufweist. Das Fahrzeug weist dann die bereits in Zusammenhang mit dem Verfahren, der Steuerschaltung bzw. der Beduftungsvorrichtung beschriebenen Eigenschaften und Vorteile in analoger Weise auf.

Im Folgenden wird die Erfindung an Hand von Ausführungsbeispielen und unter Bezugnahme auf die beigefügten Zeichnungen näher beschrieben. Es zeigen:
- Fig. 1:: Eine schematische Ansicht eines möglichen Ausführungsbeispiels einer Beduftungsvorrichtung;
- Fig. 2:: ein Übersichtsschema bezüglich der Aufbereitung eines Ansteuersignals für die Beduftungsvorrichtung;
- Fig. 3:: ein erstes Beispiel für die Ansteuerung einer Beduftungsvorrichtung;
- Fig. 4:: ein zweites Beispiel für die Ansteuerung einer Beduftungsvorrichtung;
- Fig. 5:: ein drittes Beispiel für die Ansteuerung einer Beduftungsvorrichtung.

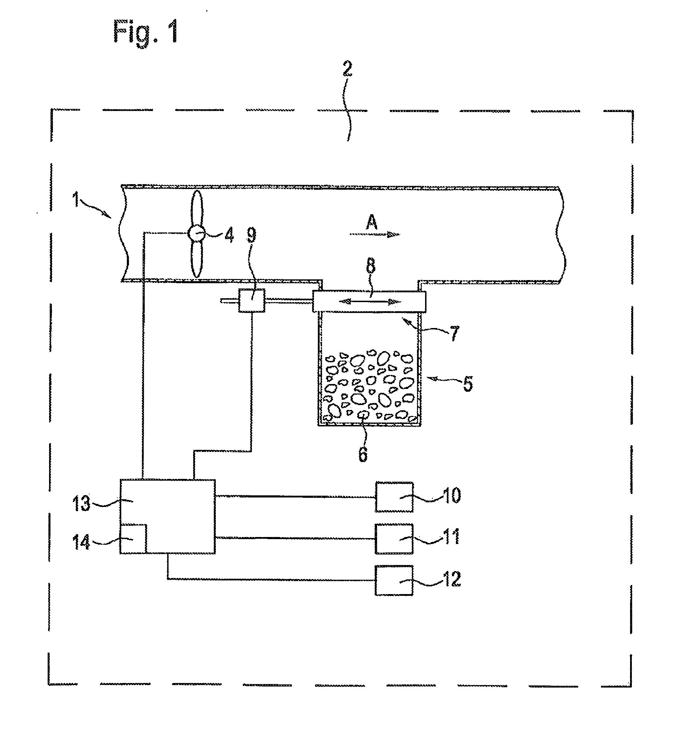

In Fig. 1 ist in einem schematischen Übersichtsplan eine Beduftungsvorrichtung 1 dargestellt, welche beispielsweise in ein vorliegend nur schematisch angedeutetes Automobil 2 eingebaut werden kann, um den Fahrzeuginnenraum des Automobils 2 zu beduften. Die Beduftungsvorrichtung 1 weist einen Luftkanal 3 auf, durch den ein Luftstrom A hindurchströmt. Der Luftstrom A wird von einem Gebläse 4 erzeugt. Die Beduftungsvorrichtung 1 kann als Teil der Kraftfahrzeugklimaanlage des Automobils 2 ausgebildet sein. Ebenso ist es möglich, dass die Beduftungsvorrichtung 1 als separate Einrichtung im Automobil 2 vorgesehen ist.

Seitlich des Luftkanals 3 der Beduftungsvorrichtung 1 befindet sich ein Duftmittelbehälter 5, in dem sich ein Duftmittel 6 befindet. Im vorliegend dargestellten Ausführungsbeispiel ist das Duftmittel 6 als ein mit einem Duftmittel versehenes Granulat ausgebildet. Zwischen dem Duftmittelbehälter 5 und dem Duftkanal 3 ist eine Verbindungsöffnung 7 vorgesehen. Die Verbindungsöffnung 7 kann durch einen Verschlussdeckel 8 geöffnet bzw. verschlossen werden. Der Verschlussdeckel 8 wird im vorliegend dargestellten Ausführungsbeispiel von einem Stellmotor 9 bewegt. Je nach Stellung des Verschlussdeckels 8 ist die Verbindungsöffnung 7 zwischen Duftmittelbehälter 5 und Luftkanal 3 mehr oder weniger weit geöffnet, so dass der durch die Beduftungsvorrichtung 1 strömende Luftstrom A stärker, schwächer oder gar nicht beduftet wird.

Die Funktion der Beduftungsvorrichtung 1 wird durch eine elektronische Steuerung 13 gesteuert. Die elektronische Steuerung 13 umfasst neben einer entsprechenden Logikschaltung insbesondere ein Speicherelement 14, in dem die unterschiedlichen Korrekturkurven bzw. Korrekturalgorithmen gespeichert sind. Die elektronische Steuerung 13 ist mit dem Stellmotor 9 und dem Gebläse 4 verbunden, und steuert deren Funktion.

Weiterhin ist die elektronische Steuerung 13 mit unterschiedlichen Bedienelementen 10, welche im Automobil 2 angeordnet sind, verbunden. Hier kann der Benutzer des Automobils 2 unterschiedliche Einstellungen vornehmen. Außerdem ist die elektronische Steuerung 13 mit Einstellelementen 11 verbunden, die jedoch nur für den Hersteller des Automobils 2, bzw. für eine Werkstätte zugänglich sind. Hier können diverse Grundeinstellungen, wie beispielsweise eine Ländervoreinstellung, vorgenommen werden. Darüber hinaus ist die elektronische Steuerung 13 noch mit Sensoren 12 verbunden. Mit Hilfe der Sensoren 12 kann die elektronische Steuerung beispielsweise Daten bezüglich der Außentemperatur, bezüglich der Luftfeuchte, bezüglich des Sonneneinfalls und dergleichen erhalten. Obgleich vorliegend jeweils nur ein Bedienelement 10, ein Einstellelement 11 bzw. ein Sensor 12 dargestellt sind, ist es selbstverständlich auch möglich, dass hier gegebenenfalls jeweils eine Mehrzahl an entsprechenden Einrichtungen 10, 11, 12 vorgesehen wird.

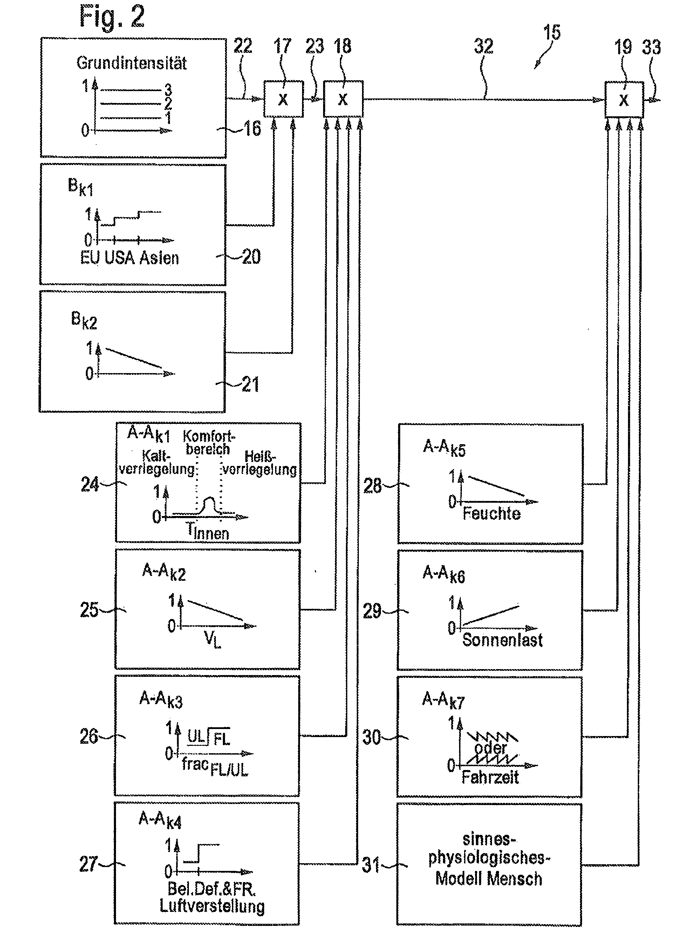

In Fig. 2 ist ein schematisches Diagramm 15 dargestellt, welches ein Ausführungsbeispiel dafür zeigt, wie ein Ansteuersignal 33 für die Beduftungsvorrichtung 1, insbesondere für den Stellmotor 9 des Verschlussdeckels 8 und/oder das Gebläse 4, aufbereitet wird. Weiterhin sind im Diagramm 15 der Fig. 2 diverse unterschiedliche Korrekturfunktionen qualitativ angedeutet.

Zunächst wird ein Wert 22 für die Grundintensität 16 ermittelt, Dazu fragt die elektronische Steuerung 13 beispielsweise das Bedienelement 10 ab. Im vorliegend dargestellten Ausführungsbeispiel kann der Benutzer des Automobils 2 die Duftintensität 16 ausschalten, bzw. drei unterschiedliche Stärken (schwach = 1, mittel = 2, stark = 3) auswählen.

Diese Information 22 wird einem ersten Signalprozessor 17 der elektronischen Steuerung 13 zugeführt. Weiterhin wird dem ersten Signalprozessor 17 ein erster Korrekturwert in Form einer Länderkorrektur 20 zugeführt. Bei der Länderkorrektur 20 handelt es sich um eine Basiskorrektur. Der entsprechende Wert wird beispielsweise vom Einstellelement 11, mit dem Werksvoreinstellungen für das Automobil 2 getroffen werden, ausgelesen. Im vorliegend dargestellten Ausführungsbeispiel sind für unterschiedliche Regionen (längs der Abszisse dargestellt) unterschiedliche Korrekturfaktoren Bₖ₁ längs der Ordinate dargestellt.

Weiterhin wird der Wert 22 für die Grundintensität 16 im ersten Signalprozessor 17 um eine zweite Basiskorrektur korrigiert, welche den Wert der Grundintensität 16 hinsichtlich der Außenluftgüte 21 korrigiert. Mit zunehmender Außenluftgüte ALG (längs der Abszisse dargestellt; "bessere" Luft) kann die erforderliche Beduftung des Luftstroms A schwächer als bei niedriger Außenluftgüte ALG ("schlechter" Luft) geringer ausfallen. Dementsprechend wird das Grundsignal 22 um einen Korrekturwert Bₖ₂ korrigiert.

Nach Durchführung der Basiskorrekturen im ersten Signalprozessor 17 liegt das erste Zwischensignal 23 vor. Es sollte darauf hingewiesen werden, dass das erste Zwischensignal 23 (also das Grundsignal 22 nach Durchführung der Basiskorrektur im ersten Signalprozessor 17) unabhängig davon verwendet werden kann, ob die Beduftung von den Fahrzeugpassagieren dauerhaft wahrgenommen werden soll, oder nicht. Denn auch wenn der Innenraum des Automobils 2 nach einiger Zeit für die Insassen "geruchsfrei" wirken soll, so ist es beim Vorhandensein übler Außengerüche erforderlich, eine hohe Anfangsbeduftungsintensität vorzusehen, um den üblen Geruch der Außenluft zu überdecken.

Das erste Zwischensignal 23 wird anschließend einem zweiten Signalprozessor 18 übergeben. Im zweiten Signalprozessor 18 wird eine erste Anti-Adaptionskorrektur vorgenommen. Im vorliegend dargestellten Ausführungsbeispiel erfolgt im zweiten Signalprozessor 18 eine Korrektur bezüglich der Innenraumtemperatur T_{Innen} 24, der Luftmenge V_{L} 25 dem Verhältnis zwischen Frischluft und Umluft Frac_{FL/UL} 26 (kann aus der Stellung der Einstellklappe zwischen Umluftanteil und Frischluftanteil ermittelt werden) und der Luftverteilung 27, welche die Aufteilung der in den Fahrzeuginnenraum des Automobils 2 eingeblasenen Luft auf die Defrosterdüsen, die Frischluftdüsen und die Fußraumdüsen angibt.

Bei der Innenraumtemperaturkorrektur 24 erfolgt eine Korrektur ausschließlich, wenn sich die Innenraumtemperatur T_{Innen} in einem Komfortbereich befindet. Demgegenüber erfolgt bei sehr niedrigen und sehr hohen Innenraumtemperaturen keine Beduftung, da bei derartigen Temperaturen T_{Innen} der Komfort für die Fahrzeuginsassen ohnehin stark eingeschränkt ist. Bei der Luftmengenkorrektur wird mit zunehmender Luftmenge V_{L} die Beduftungsintensität zu niedrigeren Duftintensitäten hin verändert. Denn wenn der Fahrzeuginsasse eine große Luftmenge abruft, so deutet dies darauf hin, dass er frische, unverfälschte Luft zu erhalten wünscht.

Bei der Frischluft-zu-Umluftkorrektur 26 erfolgt eine stärkere Beduftung, wenn der Frischluftanteil hoch gewählt wird, da hier die Duftstoffe nicht innerhalb des Fahrzeuginneren zirkulieren, sondern stets nach außen abgeführt werden, und daher mit der zugeführten Frischluft neu eingebracht werden müssen. Bei der Luftverteilkorrektur 27 erfolgt eine höhere Beduftungsintensiät, wenn der Luftaustritt vornehmlich im Bereich der Defrosterdüse (Def) und der Frischluftdüse (FR) erfolgt, da dies darauf hindeutet, dass der Fahrer mit Duftstoffen versorgt zu werden wünscht.

Nach Durchführung des ersten Satzes von Anti-Adaptionskorrekturen im zweiten Signalprozessor 18 erhält man das zweite Zwischensignal 32, welches als Eingabesignal für den dritten Signalprozessor 19 dient.

Im dritten Signalprozessor 19 erfolgt der zweite Teil der Anti-Adaptionskorrekturen, im vorliegend dargestellten Beispiel die Luftfeuchtigkeitskorrektur 28, die Sonnenlastkorrektur 29, die Fahrzeitkorrektur 30 und die sinnesphysiologische Einflusskorrektur 31.

Es hat sich herausgestellt, dass bei höherer Luftfeuchtigkeit die Beduftung vorteilhafterweise verringert wird. Dementsprechend ist im Zusammenhang mit der Luftfeuchtigkeitskorrektur 28 eine mögliche, geeignete Korrektur angedeutet. Weiterhin hat sich als sinnvoll erwiesen, bei hoher Sonnenlast eine verstärkte Beduftung vorzunehmen. Auch dies ist in Fig. 2 dem Funktionsschema der Sonnenlastkorrektur 29 zu entnehmen. Die Fahrzeitkorrektur 30 (gegebenenfalls auch eine Betriebszeitkorrektur bezüglich der Betriebszeit der Beduftungsvorrichtung 1) erfolgt sinnvollerweise als Sägezahnkurve, wie Versuche ergeben haben. Die Sägezahnkurve kann entweder mit einer ansteigenden Flanke oder mit einer abfallenden Flanke realisiert werden. In beiden Fällen kann sich ein für die Fahrzeuginsassen besonders langanhaltender, gegebenenfalls auch gleichmäßiger Dufteindruck ergeben, ohne dass die Sinneswahrnehmung nach einer gewissen Zeitspanne entfällt.

Bei der sinnesphysiologischen Einflusskorrektur 31 werden vorzugsweise experimentelle Daten verwendet, welche der medizinischen Fachliteratur entnommen werden können.

Nach Durchführung der zweiten Anti-Adaptionskorrektur im dritten Signalprozessor 19 wird das Ausgabesignal 33 beispielsweise an das Gebläse 4 und/oder den Stellmotor 9 der Beduftungsvorrichtung 1 weitergeleitet.

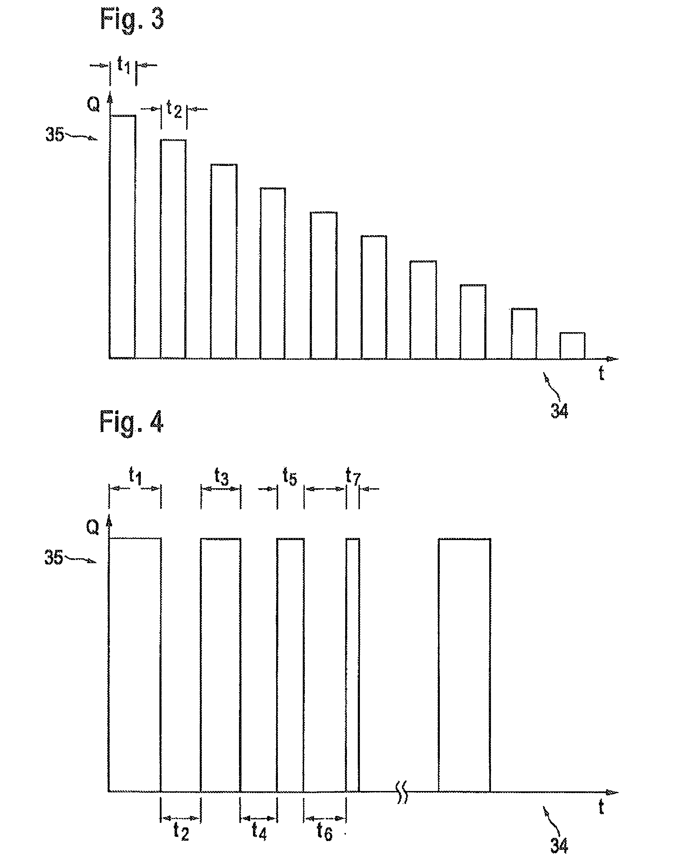

In den Fig. 3 bis 5 sind unterschiedliche Ansteuerungsmöglichkeiten für die Beduftungsvorrichtung 1 schematisch dargestellt, welche jeweils dazu geeignet sind, einen Dauergewöhnungseffekt an die Duftstoffe zu verhindern, und darüber hinaus zur Realisierung der unterschiedlichen Korrekturen (Basiskorrektur/Anti-Adaptionskorrektur) geeignet sind.

Beispielsweise ist es möglich, in fixen Zeitintervallen (fix sowohl hinsichtlich der Öffnungsdauer des Verschlussdeckels 8, als auch fix hinsichtlich der Verschlussdauer des Verschlussdeckels 8) eine Variation vorzunehmen, indem der Öffnungsquerschnitt Q in unterschiedlichen Zeitintervallen unterschiedlich groß gewählt wird. Dies ist in Fig. 3 angedeutet, in der längs der Abszisse 34 die Zeit und längs der Ordinate 35 der Öffnungsquerschnitt Q dargestellt sind.

Zusätzlich oder alternativ ist es auch möglich, die Intervalle, in denen der Verschlussdeckel 8 geöffnet ist (T₁, T₃, T₅, T₇ ...), bzw. in denen der Verschlussdeckel 8 geschlossen ist (T₂, T₄, T₆ ...) zu variieren. Dabei ist es nicht unbedingt erforderlich sowohl die Aufzeit, als auch die Zuzeit zu verändern. Es kann sich als ausreichend erweisen, lediglich die Aufzeit bzw. lediglich die Zuzeit zu variieren. Dies ist in Fig. 4 dargestellt. Nach einer gewissen Zeit kann sich das Intervallschema auch wiederholen. Beim in Fig. 4 dargestellten Ausführungsbeispiel ist der Öffnungsquerschnitt Q (längs der Ordinate 35 aufgetragen) jeweils konstant gewählt.

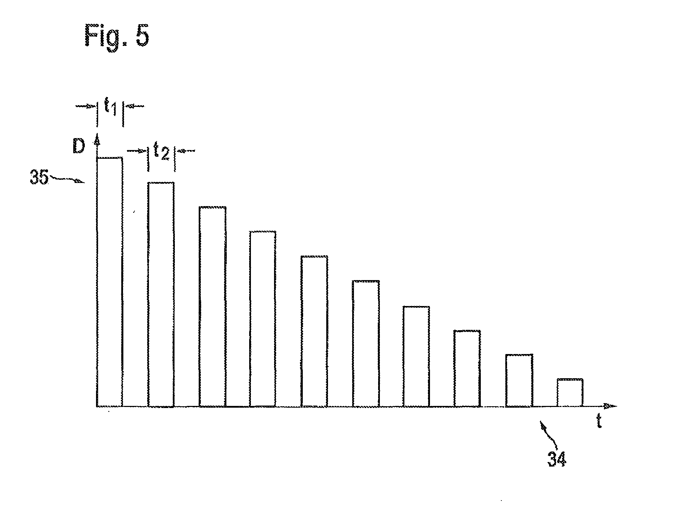

Ebenfalls zusätzlich oder alternativ ist es möglich, die Lüfterdrehzahl D zu variieren, was in Fig. 5 angedeutet ist. Beim in Fig. 5 dargestellten Ausführungsbeispiel sind die jeweiligen Anzeiten bzw. Auszeiten des Lüfters 4 konstant gewählt. Analog zur Fig. 4 ist es jedoch zusätzlich oder alternativ denkbar, die Zeitintervalle zu variieren.

## Patentansprüche

1. Verfahren (15) zur Steuerung der Duftintensität bei einer Beduftungsvorrichtung (1), wobei zumindest eine Grundstellwertvorgabe (16, 22) durch zumindest eine Korrekturfunktion (20, 21, 24, 25, 26, 27, 28, 29, 30, 31) beeinflusst wird, **dadurch gekennzeichnet, dass** eine Mehrzahl an Korrekturfunktionen (20, 21, 24, 25, 26, 27, 28, 29, 30, 31) verwendet wird, wobei eine Korrekturfunktion (20, 21, 24, 25, 26, 27, 28, 29, 30, 31) als Basiskorrekturfunktion (20, 21) und eine Korrekturfunktion als Anti-Adaptionskorrekturfunktion (24, 25, 26, 27, 28, 29, 30, 31) ausgeführt ist, wobei eine Anti-Adaptionskorrekturfunktion eine Korrekturfunktion ist, die die dauerhafte Wahrnehmbarkeit der freigesetzten Duftstoffe für die Benutzer sicher stellt.

2. Verfahren (15) nach Anspruch 1, **dadurch gekennzeichnet, dass** wenigstens eine Korrekturfunktion (20, 21, 24, 25, 26, 27, 28, 29, 30, 31) der Gruppe entnommen ist, die länderspezifische Korrekturfunktionen (20), Außentuftkorrekturfunktionen (21), Kabinentemperaturkorrekturfunktionen (24), Luftmengenkorrekturfunktionen (25), Umluft-/Frischluftauswahlkorrekturfunktionen (26), Luftverteilungskorrekturfunktionen (27), Luftfeuchtekorrekturfunktionen (28), Sonnenlastkorrekturfunktionen (29), Fahrzeitkorrekturtunktionen (30), Betriebszeitkorrekturen, sinnesphysiologische Korrekturfunktionen (31), Uhrzeit-korrekturfunktionen, Fahrgeschwindigkeitskorrekturfunktionen und Fahrzeugbesetzungskorrekturfunktionen umfasst.

3. Verfahren (15) nach einem der Ansprüche 1 bis 2, **dadurch gekennzeichnet, dass** zumindest eine Grundstellwertvorgabe (16, 22) die vom Bediener eingestellte Beduftungsintensität umfasst.

4. Verfahren (15) nach einem der Ansprüche 1 bis 3, **dadurch gekennzeichnet, dass** zumindest eine Korrekturfunktion (20, 21, 24, 25, 26, 27, 28, 29, 30, 31) zumindest bereichsweise als analytische Funktion hinterlegt ist.

5. Verfahren (15) nach einem der Ansprüche 1 bis 4, **dadurch gekennzeichnet, dass** zumindest eine Korrekturfunktion (20, 21, 24, 25, 26, 27, 28, 29, 30, 31) zumindest bereichsweise als Wertetabelle hinterlegt ist.

6. Verfahren (15) nach einem der Ansprüche 1 bis 5, **dadurch gekennzeichnet, dass** eine Änderung der Duftintensität zumindest zeitweise und/oder zumindest teilweise durch Änderung der freigesetzten Duftmenge (8, Fig. 3), Änderung des Luftvolumens (4, Fig. 5), Änderung der Beduftungsintervalle (Fig. 4) und/oder Änderung der beduftungsfreien Intervalle (Fig. 4) erfalgt.

7. Verfahren (15) nach einem der Ansprüche 1 bis 6, **dadurch gekennzeichnet, dass** ein Fahrzeug (2), insbesondere ein Kraftfahrzeug (2), vorzugsweise ein kraftbetriebenes Landfahrzeug (2) beduftet wird.

8. Steuerschaltung (13) für eine Beduftungsvorrichtung (1), welche derart ausgebildet und eingerichtet ist, dass sie ein Verfahren (15) gemäß einem der Ansprüche 1 bis 7 durchführen kann.

9. Steuerschaltung (13) nach Anspruch 8, **gekennzeichnet durch** wenigstens eine Speichereinrichtung (14), insbesondere wenigstens eine elektronische Speichereinrichtung (14), in welcher zumindest eine Korrekturfunktion (20, 21, 24, 25, 26, 27, 28, 29, 30, 31) gespeichert ist.

10. Beduftungsvorrichtung (1), für ein Fahrzeug (2), insbesondere für ein Kraftfahrzeug (2), vorzugsweise für ein kraftbetriebenes Landfahrzeug (2) aufweisend zumindest eine Steuerschaltung (13) nach Anspruch 8 oder 9.

11. Fahrzeug (2), vorzugsweise ein kraftbetriebenes Landfahrzeug (2), aufweisend zumindest eine Beduftungsvorrichtung (1) nach Anspruch 10 und/oder zumindest eine Steuerschaltung (13) nach Anspruch 8 oder 9.

## Claims

1. A method (15) for controlling the fragrance intensity of an odorising device (1), wherein at least one basic control value parameter (16, 22) is influenced by at least one correction function (20, 21, 24, 25, 26, 27, 28, 29, 30, 31), **characterised in that** a plurality of correction functions (20, 21, 24, 25, 26, 27, 28, 29, 30, 31) are used, wherein one correction function (20, 21, 24, 25, 26, 27, 28, 29, 30, 31) is designed as a basic correction function (20, 21) and one correction function is designed as an anti-adaptation correction function (24, 25, 26, 27, 28, 29, 30, 31), wherein an anti-adaptation function is a correction function which ensures that the user perceives the released fragrances over a lasting period.

2. The method (15) according to claim 1, **characterised in that** at least one correction function (20, 21, 24, 25, 26, 27, 28, 29, 30, 31) is taken from the group comprising country-specific correction functions (20), ambient air correction functions (21), interior temperature correction functions (24), air volume correction functions (25), recirculating air/fresh air selection correction functions (26), air distribution correction functions (27), air moisture correction functions (28), solar load correction functions (29), journey time correction functions (30), operating time corrections, sensory physiological correction functions (31), time correction functions, travel speed correction functions and vehicle occupation correction functions.

3. The method (15) according to one of claims 1 to 2, **characterised in that** at least one basic control value parameter (16, 22) comprises the odorising intensity set by the operator.

4. The method (15) according to one of claims 1 to 3, **characterised in that** at least one correction function (20, 21, 24, 25, 26, 27, 28, 29, 30, 31) is stored, at least in areas, as an analytical function.

5. The method (15) according to one of claims 1 to 4, **characterised in that** at least one correction function (20, 21, 24, 25, 26, 27, 28, 29, 30, 31) is stored, at least in areas, as a value table.

6. The method (15) according to one of claims 1 to 5, **characterised in that** the fragrance intensity is changed, at least temporarily and/or at least in part, by changing the amount of released fragrance (8, figure 3), changing the air volume (4, figure 5), changing the odorising intervals (figure 4) and/or changing the odorising-free intervals (figure 4).

7. The method (15) according to one of claims 1, to 6, **characterised in that** a vehicle (2), in particular a motor vehicle (2), preferably a power-driven ground vehicle (2), is odorised.

8. A control circuit (13) a for an odorising device (1), said circuit being designed and adapted in such a way that it can carry out a method (15) according to one of claims 1 to 7.

9. The control circuit (13) according to claim 8, **characterised by** at least one memory unit (14), in particular at least one electronic memory unit (14), in which at least one correction function (20, 21, 24, 25, 26, 27, 28, 29, 30, 31) is stored.

10. An odorising devise (1) for a vehicle (2), in particular for a motor vehicle (2), preferably for a power-driven ground vehicle (2), comprising at least one control circuit (13) according to claim 8 or 9.

11. A vehicle (2), preferably a power-driven ground vehicle (2), comprising at least one odorising device (1) according to Claim 10 and/or at least one control circuit (13) according to claim 8 or 9.

## Revendications

1. Procédé (15) de commande de l'intensité de parfum concernant un dispositif (1) servant à parfumer, où au moins une prédéfinition des valeurs de réglage de base (16, 22) est influencée par au moins une fonction de correction (20, 21, 24, 25, 26, 27, 28, 29, 30, 31), **caractérisé en ce qu'**une pluralité de fonctions de correction (20, 21, 24, 25, 26, 27, 28, 29, 30, 31) est utilisés, où une fonction de correction (20, 21, 24, 25, 26, 27, 28, 29, 30, 31) est exécutée comme une fonction de correction de base (20, 21), une fonction de correction étant exécutée comme une fonction de correction d'anti-adaptation (24, 25, 26, 27, 28, 29, 30, 31), où une fonction de correction d'anti-adaptation est une fonction de correction qui préserve, pour les utilisateurs, la perceptibilité permanente des parfums dégagés.

2. Procédé (15) selon la revendication 1, **caractérisé en ce qu'**au moins une fonction de correction (20, 21, 24, 25, 26, 27, 28, 29, 30, 31) est extraite du groupe qui comprend des fonctions de correction spécifiques aux pays (20), des fonctions de correction d'air extérieur (21), des fonctions de correction des températures de cabines (24), des fonctions de correction de débit d'air (25), des fonctions de correction de sélection entre air recyclé/air frais (26), des fonctions de correction de la répartition d'air (27), des fonctions de correction d'humidité de l'air (28), des fonctions de correction de l'importance de l'ensoleillement (29), des fonctions de correction de temps de conduite (30), des corrections de temps de fonctionnement, des fonctions de correction neurophysiologiques (31), des fonctions de correction d'heure, des fonctions de correction de vitesse de roulage et des fonctions de correction d'occupation du véhicule.

3. Procédé (15) selon l'une des revendications 1 à 2, **caractérisé en ce qu'**au moins une prédéfinition des valeurs de réglage de base (16, 22) comprend l'intensité de parfumage réglée par l'utilisateur.

4. Procédé (15) selon l'une quelconque des revendications 1 à 3, **caractérisé en ce qu'**au moins une fonction de correction (20, 21, 24, 25, 26, 27, 28, 29, 30, 31) est mémorisée au moins partiellement comme fonction analytique.

5. Procédé (15) selon l'une quelconque des revendications 1 à 4, **caractérisé en ce qu'**au moins une fonction de correction (20, 21, 24, 25, 26, 27, 28, 29, 30, 31) est mémorisée au moins partiellement comme tableau des valeurs.

6. Procédé (15) selon l'une quelconque des revendications 1 à 5, **caractérisé en ce qu'**une modification de l'intensité de parfum se produit au moins temporairement et/ou au moins partiellement par modification de la quantité de parfum dégagée (8, figure 3), par modification du volume d'air (4, figure 5), par modification des intervalles de parfumage (figure 4) et/ou par modification des intervalles sans parfumage (figure 4).

7. Procédé (15) selon l'une quelconque des revendications 1 à 6, **caractérisé en ce qu'**un véhicule (2), en particulier un véhicule automobile (2), de préférence un véhicule routier à moteur (2), est parfumé.

8. Circuit de commande (13) pour un dispositif (1) servant à parfumer qui est configuré et conçu de manière telle, qu'il puisse exécuter un procécé (15) conformément à l'une quelconque des revendications 1 à 7.

9. Circuit de commande (13) selon la revendication 8, **caractérisé par** au moins un dispositif de mémoire (14), en particulier au moins un dispositif de mémoire électronique (14) dans lequel est mémorisée au moins une fonction de correction (20, 21, 24, 25, 26, 27, 28, 29, 30, 31).

10. Dispositif (1) servant à parfumer, pour un véhicule (2), en particulier pour un véhicule automobile (2), de préférence pour un véhicule routier à moteurs présentant au moins un circuit de commande (13) selon la revendication 8 ou 9.

11. Véhicule (2), de préférence un véhicule routier à moteur (2), présentant au moins un dispositif (1) servant à parfumer selon la revendications 10 et/ou au moins un circuit de commande (13) selon la revendication 8 ou 9.
